(19) Europäisches Patentamt
European Patent Office
Office européen des brevets

(11) Numéro de publication : **0 586 295 A1**

(12) **DEMANDE DE BREVET EUROPEEN**

(21) Numéro de dépôt : **93402121.3**

(22) Date de dépôt : **31.08.93**

(51) Int. Cl.$^5$ : **A61K 7/00,** A61K 7/15, C09K 3/30

(30) Priorité : **31.08.92 FR 9210419**

(43) Date de publication de la demande :
**09.03.94 Bulletin 94/10**

(84) Etats contractants désignés :
**AT BE CH DE DK ES FR GB GR IE IT LI LU MC NL PT SE**

(71) Demandeur : **NLN**
**Z.I.La Vigne-aux-Loups 18, Avenue Arago, B.P.28**
**F-91382 Chilly-Mazarin (FR)**

(72) Inventeur : **Goffinet, Pierre Charles Emile**
**22 allée des Petites Garennes**
**F-91190 Gif sur Yvette (FR)**
Inventeur : **Le Nigen, Norbert Claude**
**14 rue des Saussaies**
**F-75008 Paris (FR)**

(74) Mandataire : **Le Roux, Martine et al**
**Cabinet Beau de Loménie 158, rue de l'Université**
**F-75340 Paris Cédex 07 (FR)**

(54) **Compositions fluides, rapidement moussantes, exemptes de savons, contenant un hydrocarbure inférieur et propulsées en récipient sous pression de gaz comprimé.**

(57) L'invention concerne une composition fluide, rapidement moussante propulsée en récipient sous pression de gaz comprimé, caractérisée en ce qu'elle comprend, en pourcentages pondéraux :

a) de 0,01% à 40% d'un agent tensioactif moussant anionique, non ionique, cationique, zwitterionique ou amphotère, dont la tension superficielle d'une solution aqueuse à 0,1% enregistre un abaissement d'au moins 30 mN/m par rapport à la tension superficielle de l'eau pure à 20°C,

b) de 1% à 10% d'un hydrocarbure en $C_5$, saturé ou mono- ou polyinsaturé, liquide à la température ambiante et à la pression atmosphérique, et ayant un point d'ébullition inférieur à 50°C, sous une pression de 100 kPa (1 bar),

c) de 0,01% à 30% d'une matière active, le complément à 100% étant constitué par de l'eau ;

et dans laquelle la proportion de savons d'acides gras saturés de longueur de chaîne supérieure à 12 atomes de carbone, lorsqu'ils sont présents, ne peut excéder 0,5%, ladite composition étant fluide à la température ambiante et ayant de préférence une viscosité à 25°C inférieure à 0,5 Pa.s (500 cP).

Cette composition peut être notamment utilisée en tant que produit d'entretien, produit phytosanitaire, produit pharmaceutique ou produit cosmétique.

EP 0 586 295 A1

L'invention concerne des compositions fluides, rapidement moussantes, exemptes de savons, contenant un hydrocarbure inférieur et propulsées en récipient sous pression de gaz comprimé.

Les récipients sous pression de gaz comprimé utilisables dans le cadre de l'invention peuvent être soit des générateurs d'aérosols, par exemple métalliques, étanches et mis industriellement sous pression d'un gaz non liquéfié, soit des récipients dont la mise en pression est effectuée régulièrement par l'utilisateur à l'aide d'un système de pompe manuelle à piston, par exemple les systèmes AIR-SPRAY® commercialisés par la Société du même nom.

Les générateurs d'aérosols communément utilisés sont soit des aérosols à gaz liquéfié, soit des aérosols à gaz comprimé ou partiellement dissout.

Les aérosols à gaz liquéfié comprennent un mélange intime d'une composition à propulser et d'un gaz propulseur sous forme liquide. La pression est essentiellement déterminée par la pression de vapeur saturante du gaz propulseur liquéfié et est donc sensiblement constante tout au long de la durée de l'aérosol. En sortie de valve, le gaz liquéfié se détend brutalement en passant à l'état gazeux et atomise les particules liquides en leur conférant une taille faible évitant les retombées (le diamètre des particules est d'environ 10 à 40 μm).

Les gaz habituellement utilisés dans ce type d'aérosols sont notamment des hydrocarbures chlorofluorés (CFC), des mélanges d'alcanes à chaînes courtes (par exemple n-butane, isobutane ou propane) ou du diméthyléther (DME).

Néanmoins, ce type d'agent propulseur présente de nombreux inconvénients, notamment sur le plan écologique.

En effet, la dégradation des CFC dans la haute atmosphère sous l'action de rayons ultraviolets génère du chlore radicalaire et contribue à la diminution de la couche d'ozone, rendant l'utilisation de ces composés indésirable.

Par ailleurs,les propulseurs gazeux du type des alcanes inférieurs, le DME et le $CO_2$, classiquement utilisés comme substituts des CFC, sont sans effet sur la couche d'ozone mais soit contribuent, par réaction avec les oxydes d'azote NOx, à la formation de gaz très irritants et susceptibles de générer de graves problèmes respiratoires au-dessus des zones de forte densité d'émission (cas du DME et des alcanes légers), soit participent , directement ou après dégradation photochimique, à freiner la ré-émission du rayonnement infra-rouge qui atteint la surface de la terre, conduisant à une augmentation de la température moyenne de surface (phénomène d'effet de serre).Ces phénomènes peuvent avoir des conséquences néfastes sur le plan climatologique et écologique.

D'autre part, l'utilisation dans des fortes proportions (>20 %) d'alcanes inférieurs dont le point d'ébullition est inférieur à la température ambiante ou de DME, très inflammables, est susceptible d'entraîner des risques pour l'utilisateur dans des conditions inhabituelles d'utilisation, et implique au niveau de la production la mise en place d'installations coûteuses afin d'en assurer la sécurité.

Les systèmes générateurs d'aérosols sous pression de gaz comprimés présentent un certain nombre d'avantages par rapport aux générateurs d'aérosols utilisant des gaz liquéfiés, notamment la possibilité d'utiliser des gaz inexplosibles, ininflammables et sans effets indésirables sur le plan écologique tels que l'air, l'azote, l'oxygène, l'hélium et les gaz rares, ainsi que leurs mélanges.

Toutefois, les systèmes générateurs d'aérosols à gaz comprimés ne permettaient pas jusqu'à présent, du fait de leur principe même (séparation des phases gaz/liquide en leur sein), de générer des mousses stables, lesquelles impliquent l'incorporation, sous forme notamment d'émulsion, de microémulsion ou de solution, d'importantes quantités de gaz dans la composition. C'est en effet la détente de ce gaz lors de sa vaporisation à la mise à la pression atmosphérique qui, combinée avec la présence d'un agent tensioactif, génère une mousse stable.

L'incorporation de ces quantités relativement importantes de gaz dans la composition se fait le plus souvent grâce à l'utilisation de gaz liquéfié, de préférence émulsionné de manière stable au sein de la composition. Cette incorporation peut également être obtenue par la solubilisation dans les compositions de gaz très solubles dans les mélanges aqueux, hydroalcooliques ou eau + éther tels que le $CO_2$ ou le $N_2O$.On obtient ainsi des émulsions stables de $N_2O$ dans des compositions alimentaires telles que la crème Chantilly.

On notera que l'utilisation des gaz liquéfiés est aisée, et permet de plus de maintenir une pression constante dans le boîtier.

Le brevet WO 91/07 943 mentionne l'utilisation de mélanges d'alcanes inférieurs comme agents formateurs de mousse dans des aérosols à gaz liquéfiés.

Les gaz comprimés à faible solubilité dans les compositions aqueuses, tels que l'air, les gaz rares, l'azote, etc., agissent par effet de piston et, n'étant que faiblement dissous ou émulsionnés dans la composition, ne peuvent par eux-mêmes générer des mousses stables.

L'utilisation d'alcanes inférieurs à la fois en tant que gaz propulseurs et agents formant une mousse à post-expansion (par application sur la peau) dans des formulations de gels de rasage est décrite dans le brevet GB

2 166 150.

Par ailleurs, le brevet GB 1 444 334 mentionne cette même utilisation en indiquant que les gaz propulseurs peuvent être des gaz inertes comprimés tels que l'azote, l'argon, etc.

Ces compositions ont toutes pour particularité de se présenter sous forme d'un gel stable et de haute viscosité substantiellement non moussant à la température ambiante, qui ne se transforme en mousse crémeuse que par application, étalement et manipulation, notamment sur la peau.

Cette progressivité de montée en mousse associée à la nécessité d'une action mécanique est notamment liée à la haute viscosité des compositions considérées qui contiennent d'assez fortes proportions de savons ; ces caractéristiques peuvent être souhaitables pour certains types de produits de rasage, mais sont hautement indésirables dans de nombreux autres cas, et notamment celui des produits d'entretien moussants (nettoyage des fours, moquettes, sanitaires, meubles, etc.), cosmétiques (mousses coiffantes, traitantes, shampooings, etc.) ou phytosanitaires (herbicides) pour lesquels une mousse immédiate ou à montée rapide est un élément essentiel de la performance objective et/ou de la perception qualitative.

De manière surprenante, on a maintenant trouvé que l'incorporation d'un hydrocarbure en $C_5$, saturé ou mono- ou polyinsaturé, liquide à température ambiante (20°C) et à la pression atmosphérique, et ayant un point d'ébullition inférieur à 50°C dans une composition fluide, notamment ayant une viscosité inférieure à 0,5 Pa.s (500 cP), propulsée par des gaz comprimés, permettait d'obtenir rapidement une mousse abondante et stable. Cet hydrocarbure est stabilisé à l'état liquide sous forme d'émulsion, dans la phase aqueuse essentiellement grâce à la pression de gaz comprimé appliquée à l'aérosol. Cette pression évite notamment un dégazage lent qui pourrait se produire à des températures supérieures à la température d'ébullition de l'hydrocarbure à la pression atmosphérique. Un autre avantage est la détente rapide en sortie de buse qui permet d'obtenir spontanément une mousse abondante sans application d'aucun effort mécanique.

L'invention concerne donc une composition fluide, rapidement moussante, propulsée en récipient sous pression de gaz comprimé, caractérisée en ce qu'elle comprend, en pourcentages pondéraux:

a) de 0,01% à 40% d'un agent tensioactif moussant anionique, non ionique, cationique, zwitterionique ou amphotère, dont la tension superficielle d'une solution aqueuse à 0,1% enregistre un abaissement d'au moins 30 mN/m par rapport à la tension superficielle de l'eau pure à 20°C,

b) de 1% à 10% d'un hydrocarbure en $C_5$, saturé ou mono- ou polyinsaturé, liquide à la température ambiante et à la pression atmosphérique, et ayant un point d'ébullition inférieur à 50°C, sous une pression de 100 kPa (1 bar),

c) de 0,01% à 30% d'une matière active,

le complément à 100% étant constitué par de l'eau ;

et dans laquelle la proportion de savons d'acides gras saturés de longueur de chaîne supérieure à 12 atomes de carbone, lorsqu'ils sont présents, ne peut excéder 0,5%, ladite composition étant fluide à la température ambiante et ayant de préférence une viscosité à 25°C inférieure à 0,5 Pa.s (500 cP).

La viscosité de la composition est mesurée au viscosimètre BROOKFIELD Synchro-Electric LVT (aiguille n°4).

Ladite composition comprend au moins 20% d'eau.

Par "rapidement moussante", on entend une composition dont la montée en mousse se fait dans un délai inférieur ou égal à 2 s et sans autre action mécanique que la pulvérisation.

En tant qu'hydrocarbure en $C_5$, saturé ou mono- ou polyinsaturé, on utilisera avantageusement le pentane, l'isopentane, le 1- ou 2-pentène, l'isoprène-((2-méthyl)1,3-butadiène) ou encore le 1- ou 2-pentyne.

Dans un aspect préféré, l'hydrocarbure en $C_5$ est le pentane ou l'isopentane, l'isopentane étant particulièrement préféré, notamment à raison de 2% à 5% dans la composition.

Dans un aspect particulièrement avantageux de l'invention, on recherche des compositions ininflammables, tant au moment de la pulvérisation qu'au moment de l'expansion de la mousse sur la surface où elle est projetée. De telles compositions comprennent au moins 40% d'eau, les autres constituants étant tels que définis ci-dessus.

On choisira l'agent tensioactif moussant utilisé dans les compositions selon l'invention parmi les agents tensioactifs anioniques neutralisés, les agents tensioactifs non ioniques, cationiques, amphotères ou zwitterioniques et leurs mélanges.

A titre d'exemple d'agents tensioactifs moussants, on pourra notamment se référer aux agents tensioactifs cités dans le brevet FR 2 345 996, ou dans l'ouvrage "Agents de surface et émulsions (les systèmes dispersés, I)" publié dans la collection GALENICA (tome 5) par F. PUISIEUX et M. SEILLER (1983 - Lavoisier édit.).

Sont toutefois exclues de l'invention les compositions comprenant des proportions significatives (> 0,5%) de savons d'acides gras saturés de longueur de chaîne supérieure à 12 atomes de carbones, lesdits savons tendant à augmenter très fortement la viscosité des compositions, notamment en présence de l'hydrocarbure inférieur.

Cette augmentation de viscosité peut aller jusqu'à la formation de pâtes ou de gels, voire jusqu'à la solidification ; elle nuit considérablement à la cinétique de montée en mousse lors de la pulvérisation de la composition. Elle peut par ailleurs être à l'origine de phénomènes de cavitation dans l'aérosol et, dans les cas extrêmes, rendre impossible la pulvérisation.

Dans tous les cas, elle rend le produit impropre aux utilisations nécessitant une mousse immédiate et abondante sans autre intervention mécanique que la pulvérisation sous pression de la composition moussante.

Les compositions de l'invention peuvent également contenir à titre de co-solvant un alcool linéaire ou ramifié comprenant de 1 à 6 atomes de carbone. A titre d'alcool comprenant de 1 à 6 carbones, on utilisera par exemple l'éthanol, l'isopropanol ou le méthanol, l'éthanol étant préféré. Un tel alcool peut intervenir jusqu'à 40% en poids dans les compositions de l'invention.

Par matière active, on entend aussi bien des matières participant directement à l'activité du produit (par exemple un composé à activité insecticide ou herbicide pour un produit phytosanitaire) que les composés auxiliaires comme par exemple les émulsifiants, les synergisants, les agents hydratants, les agents conférant des bénéfices secondaires ainsi que les co-solvants.

La matière active pourra être notamment choisie parmi des agents nettoyants, des agents cirants, des agents désinfectants, des agents séquestrants, des ions alcalino-terreux, des agents anti-taches, des agents détartrants, des filtres anti-UV, des agents conditionneurs, des résines coiffantes, des agents insecticides ou herbicides ou des solvants.

Notamment, dans un aspect avantageux, les compositions selon l'invention comprennent un agent émulsifiant permettant d'obtenir une solubilisation ou une stabilisation définitive ou partielle, dans le temps, de l'émulsion de l'hydrocarbure inférieur à faible point d'ébullition au sein desdites compositions.

Parmi ceux-ci on peut notamment citer les alcools gras oxyéthylénés de formule générale R - $(O\text{-}CH_2\text{-}CH_2)_n\text{-}OH$ dans laquelle R représente un alkyle ou un alcényle linéaire ou ramifié comprenant de 8 à 22 atomes de carbone et n= 5 à 50, ou encore des oxydes d'amines grasses ou des alcanolamides d'alcools gras.

A titre de co-solvant, les compositions selon l'invention comprendront avantageusement au moins un composé de formule (I)

$$R_1\text{-}A\text{-}(CH_2)_x\text{-}(O)_y\text{-}R_2 \qquad (I)$$

dans laquelle:

- $R_1$ représente un groupe méthyle, éthyle ou isopropyle,
- $R_2$ représente l'hydrogène ou un groupe méthyle, éthyle ou isopropyle,
- ou bien $R_1$ et $R_2$ représentent ensemble un groupement - $CH_2$ - $CH_2$-dans la formule (I) ci-dessus cyclisée,
- x = 0, 1 ou 2
- y = 0 ou 1
- A est -O-,

$$-\overset{\overset{\displaystyle O}{\|}}{C}- \text{ ou } -\overset{\overset{\displaystyle OH}{|}}{C}H-,$$

ledit co-solvant ayant un point d'ébullition compris entre environ 25°C et 200°C à la pression atmosphérique.

A titre d'exemples non limitatifs de tels co-solvants, on peut notamment citer le méthylal (diméthoxyméthane), le dioxolanne ou l'éthylèneglycol diméthyl éther(1, 2-diméthoxyéthane).

Les compositions selon l'invention peuvent être destinées à des applications variées, sous la forme de mousses pulvérisables utilisables notamment, à titre d'exemples, en tant que:

- <u>produits d'entretien</u>
  . mousses de nettoyage des fours
  . mousses de nettoyage des moquettes
  . mousses de nettoyage des sanitaires
  . produits dépoussiérants et/ou cirants pour nettoyage des meubles
  . produits de nettoyage des vitres et toutes surfaces
  . produits prédétachants pour textiles
  . produits de nettoyage pour vaisselle
- <u>produits phytosanitaires</u>
  . produits pour traitement des plantes: herbicides, insecticides, fongicides, anti-maladies
- <u>produits cosmétiques</u>
  . produits de rasage

. mousses dépilatoires
. mousses coiffantes
. shampooings
. produits solaires filtrants ou non filtrants.

- produits pharmaceutiques

Dans un de ses aspects, l'invention concerne également l'utilisation de ladite composition liquide, notamment dans les applications mentionnées ci-dessus.

Les compositions selon l'invention sont propulsées par un gaz comprimé non liquéfiable à la température ordinaire et à la pression interne de l'aérosol ou du récipient sous pression, peu soluble dans l'eau et les solvants organiques,ininflammable, non toxique, non explosible et non polluant.

En tant que gaz comprimé, on utilisera avantageusement l'air, l'azote, l'oxygène, l'hélium et les gaz rares, ou leurs mélanges.

En revanche, le dioxyde de carbone ($CO_2$) et le protoxyde d'azote ($N_2O$) sont exclus car ils sont trop solubles dans l'eau et polluants.

Des gaz particulièrement préférés sont l'air, l'azote et l'hélium.

A l'intérieur de l'aérosol, la pression est utilement comprise entre 400 et 1200 kPa (4 et 12 bars) de préférence entre 600 et 1000 kPa (6 et 10 bars). Pendant l'utilisation, cette pression diminue mais doit rester supérieure à 150 kPa (1,5 bar), de préférence supérieure à 200 kPa (2 bars), afin de garantir un vidage complet de l'aérosol.

Dans le cas d'un système de type "AIR-SPRAY®", la pression est déterminée par l'utilisateur.

Les compositions selon l'invention sont totalement compatibles avec la composition du boîtier, qu'il soit constitué de fer blanc nu ou revêtu d'un film de protection, ou d'aluminium verni.

Dans un aspect avantageux, les compositions selon l'invention sont conditionnées dans un récipient coopérant avec un dispositif générateur d'aérosol comprenant une valve à coupelle métallique sertie sur l'ouverture du boîtier.

Dans un aspect avantageux, ladite valve est de préférence choisie parmi les valves dites "à tube capillaire". A titre d'exemple, on peut citer notamment la valve Ariane réf. 18620 (Perfect-Valois) et la valve microcapillaire réf. 05903 (Valve Précision).

Dans un autre aspect préféré, on utilisera des valves de type "à bille", notamment à titre d'exemples, la valve 360° réf. 06233 (Valve Précision) ou les valves à bille réf. 72027 et 72007 (Perfect Valois). Lesdites valves peuvent, dans un aspect avantageux, être elles-mêmes coiffées par exemple, sur le téton de valve, par un bouton-poussoir. On utilisera de préférence un bouton-poussoir de type à chambre tourbillonnaire.

A titre d'exemple de tels boutons-poussoirs, on peut notamment citer de manière non limitative les boutons-poussoirs tourbillonnaires à canaux fins ayant les références suivantes:

- WANP GP 3, WAW DU 20 73 et WAX DU 2575 (Perfect - Valois);
- COSMOS 210 201-XE COSMOS 210 390-XE et COSMOS 216 901-95 (Valve Précision);
- M 4043-2307 et M 4000 Y-2115 (Reboul SMT).

Les exemples cités ci-dessus correspondent à des modes d'utilisations préférés des compositions selon l'invention. Néanmoins, lesdites compositions présentent également des propriétés avantageuses lorsqu'elles sont utilisées avec d'autres dispositifs et notamment d'autres types de valves et de boutons-poussoirs disponibles commercialement.

Dans un autre aspect intéressant, les compositions selon l'invention sont conditionnées dans un récipient, par exemple en matière plastique, muni d'un dispositif de pulvérisation coopérant avec une pompe manuelle à piston permettant la mise ou la remise en pression du récipient par l'utilisateur.

Ce type de conditionnement est notamment commercialisé par la société AIR-SPRAY sous la dénomination AIR-SPRAY®.

On préférera néanmoins des systèmes à boîtier étanche.

On notera que, avantageusement, il n'est pas nécessaire, pour obtenir une mousse possédant les propriétés d'abondance et de stabilité mentionnées plus haut, que l'alcane inférieur à faible point d'ébullition soit intimement mélangé à la composition à propulser au sein du récipient sous pression de gaz comprimé. Le mélange peut en effet se réaliser de manière statique au sein de la valve et/ou du bouton-poussoir.

Toutefois, les compositions permettant une émulsification ou une micro-émulsification de l'alcane de matière stable ou transitoire (par exemple par agitation du récipient avant l'emploi) au sein du récipient sous pression de gaz comprimé, sont préférées.

L'invention sera mieux comprise à l'aide des exemples non limitatifs ci-après:

**Exemple 1** : mousses de nettoyage pour salle de bain

| | Composition 1 % | Composition 2 % |
|---|---|---|
| Arquad DMM LD-50® (AKZO) (a) | 0,75 | 0,50 |
| Inhibiteur de corrosion | 1,25 | 1,25 |
| Lutensol LF 600® (BASF) (b) | 3,00 | 4,50 |
| Citrate trisodique | 1,00 | - |
| EDTA tétrasodique | - | 0,50 |
| Parfum | 0,50 | 0,20 |
| Rhodopol 23® (Rhone Poulenc) | 0,05 | 0,03 |
| Isopentane | 5,00 | 7,00 |
| NaOH | - | 0,15 |
| Ethanol | - | 1,00 |
| Eau déionisée | q.s.p. 100,00 | q.s.p. 100,00 |
| Viscosité (Pa.s) | $3.10^{-3}$ | $2.10^{-3}$ |
| Inflammabilité | non | non |

(a) : chlorure de benzalkonium (solution à 50%)

(b) : alcool gras en $C_{13}$-$C_{15}$ éthoxylé (10 mol d'oxyéthylène) propoxylé (5 mol d'oxypropylène)

L'inflammabilité a été testée selon le protocole du projet de norme FEA E608X décrit ci-dessous:

**Appareillage**

Règle graduée, support et pince.
Verre de montre résistant au feu, d'environ 150 mm de diamètre.
Chronomètre gradué (0,2 seconde).
Bougie.
Balance de laboratoire à 0,1 g.
Bain d'eau maintenu à 20°C ± 1°C.
Zone avec ventilation contrôlée hors courant d'air.

Le verre de montre est placé sur une surface résistant au feu dans une zone hors courant d'air qui peut être ventilée après chaque test. La règle graduée est positionnée juste derrière le verre de montre et maintenue verticalement au moyen d'un support et d'une pince.

Le positionnement de la règle est tel que son origine est au niveau de la base du verre de montre avec le plan horizontal.

**Mode opératoire**

Avant de procéder au test d'inflammabilité, il est nécessaire de mesurer le débit du récipient qui doit être étudié. Cela en accord avec la norme concernant la mesure du débit d'un récipient aérosol.

Peser le récipient et le conditionner dans le bain d'eau pendant 30 minutes.

Sur la base du débit préalablement mesuré et en accord avec les instructions du producteur, distribuer approximativement 5g de produit sur le centre du verre de montre. Moins de 5 secondes après la distribution, présenter la bougie allumée à la base de la mousse et en même temps déclencher le chronomètre.

On note si la mousse est inflammable ou ininflammable.Dans le cas où elle est inflammable, on note également la hauteur maximum de la flamme en mm au-dessus de la base du verre de montre et la durée de la flamme en seconde.

**Exemple 2** : mousses de nettoyage des fours sans soude.

| | Composition 3 % | Composition 4 % |
|---|---|---|
| Propylène glycol mono-méthyl éther | 3,00 | - |
| Ethylène glycol mono-méthyl éther | - | 4,00 |
| Lauryl éther sulfate de Na éthoxylé à 3 moles d'oxyéthylène (27%) | 3,00 | 6,00 |
| Monoéthanolamine | 6,00 | 8,00 |
| VEEGUM T® (VAN DER BUILT) (a) | 1,25 | 1,50 |
| Inhibiteur de corrosion | 1,00 | 1,00 |
| Agent bactéricide | 0,10 | 0,10 |
| Parfum | 0,60 | 0,50 |
| Métasilicate de sodium | 4,00 | - |

| | Composition 3 % | Composition 4 % |
|---|---|---|
| Disilicate de sodium | - | 6,00 |
| Isopentane | 2,00 | 5,00 |
| Eau déionisée | q.s.p. 100,00 | q.s.p. 100,00 |
| Viscosité (en Pa.s) | 0,14 | 0,175 |
| Inflammabilité | non | non |

(a) silicate double de Mg et Al (61% de $SiO_2$).

**Exemple 3**: produits de nettoyage des meubles

| | Composition 5 % | Composition 6 % |
|---|---|---|
| Emulsion de cire de Carnauba | 7,50 | 2,00 |
| Emulsion de silicones | 2,00 | 5,00 |
| Inhibiteur de corrosion | 0,50 | 0,50 |
| Oxyde de lauryl diméthyl amine (30%) | 1,00 | 0,30 |
| Isopentane | 2,50 | - |
| n-pentane | - | 5,00 |
| Conservateur | 0,20 | 0,20 |
| Alcool gras de coprah éthoxylé à 7 moles d'oxyéthylène | 0,50 | 5,00 |
| Parfum | 0,30 | 0,10 |
| Eau déionisée | q.s.p. 100,00 | q.s.p. 100,00 |
| Viscosité (Pa.s) | $1,5.10^{-3}$ | $4,0.10^{-3}$ |
| Inflammabilité | non | non |

**Exemple 4** :

On a réalisé des essais en utilisant des systèmes générateurs d'aérosols comprenant un boîtier en fer blanc de diamètre 52 mm et de hauteur 195 mm équipé d'un bouton-poussoir tourbillonnaire (référence WANP GP 3, Perfect-Valois) monté sur une valve à bille (référence 72027, Perfect-Valois), remplis d'air comprimé sous 8 500 kPa (8,5 bars) de pression. Après agitation de l'aérosol, on a pulvérisé pendant 5 s les compositions des exemples 1, 2 et 3 sur une paroi verticale en stratifié noir et on a apprécié la quantité et la stabilité de la mousse ainsi obtenue. Dans tous les cas, la mousse obtenue montre une remarquable profusion et une excellente stabilité dans le temps (>3 min).

**Exemple 5** :

On a réalisé des essais comparatifs des viscosités et vitesses de montée en mousse de compositions avec et sans savons, afin de vérifier l'effet de ceux-ci sur la performance de produits:

| Produit | Viscosité (en Pa.s) | Pulvéri-sation | Montée en mousse |
|---|---|---|---|
| Composition 1 :<br>* sans isopentane<br>* avec isopentane | <br>$3.10^{-3}$<br>$3.10^{-3}$ | <br>facile<br>facile | <br>peu de mousse instable<br>mousse immédiate stable |
| Composition 3 :<br>* sans isopentane<br>* avec isopentane<br>* avec isopentane + 3% stéarate de Na<br>– après 3 minutes<br>– après 1 heure | <br>0,156<br>0,140<br><br>>100<br>solide | <br>facile<br>facile<br><br>assez facile<br>impossible | <br>peu de mousse instable<br>mousse immédiate stable<br><br>mousse + lente stable<br> |
| Gel de rasage selon brevet WO 91/07 943 (Gillette)* | >100 | assez facile | pas de mousse immédiate |

*composition du gel de rasage(%):

| | |
|---|---|
| acide palmitique | 9,69 |
| triéthanolamine | 5,69 |
| huile minérale | 2,90 |
| 2-éthyl-1,3-hexanediol | 1,94 |
| glycérine | 1,94 |
| OLETH-20 | 1,94 |
| hydroxyéthyl cellulose | 0,39 |
| isopentane | 2,40 |
| isobutane | 0,80 |
| eau | q.s.p. 100,00 |

Les résultats montrent que les compositions exemptes d'isopentane et/ou contenant une proportion significative de savons ne permettent pas d'obtenir les propriétés avantageuses des compositions de l'invention ( fluidité, mousse immédiate et stable ).

## Revendications

1. Composition fluide, rapidement moussante propulsée en récipient sous pression de gaz comprimé, caractérisée en ce qu'elle comprend, en pourcentages pondéraux:
   a) de 0,01% à 40% d'un agent tensioactif moussant anionique, non ionique, cationique, zwitterionique ou amphotère, dont la tension superficielle d'une solution aqueuse à 0,1% enregistre un abaissement d'au moins 30 mN/m par rapport à la tension superficielle de l'eau pure à 20°C,
   b) de 1% à 10% d'un hydrocarbure en $C_5$, saturé ou mono- ou polyinsaturé, liquide à la température ambiante et à la pression atmosphérique, et ayant un point d'ébullition inférieur à 50°C, sous une pression de 100 kPa (1 bar),
   c) de 0,01% à 30% d'une matière active,
   le complément à 100% étant constitué par de l'eau ;
   et dans laquelle la proportion de savons d'acides gras saturés de longueur de chaîne supérieure à 12 atomes de carbone, lorsqu'ils sont présents, ne peut excéder 0,5%, ladite composition étant fluide à la température ambiante et ayant de préférence une viscosité à 25°C inférieure à 0,5 Pa.s (500 cP).

2. Composition selon la revendication 1, caractérisée en ce qu'elle comprend 1 % à 10 % d'un hydrocarbure en $C_5$ choisi parmi le pentane et l'isopentane.

**3.** Composition selon l'une des revendications 1 ou 2, caractérisée en ce qu'elle comprend 1 % à 10 %, de préférence de 2 % à 5 % d'isopentane.

**4.** Composition selon l'une quelconque des revendications 1 à 3, caractérisée en ce qu'elle comprend au moins 40% d'eau.

**5.** Composition selon l'une quelconque des revendications 1 à 4, caractérisée en ce qu'elle comprend un agent émulsifiant permettant d'obtenir une solubilisation ou une stabilisation définitive ou partielle, dans le temps, de l'émulsion d'hydrocarbure en $C_5$.

**6.** Composition selon l'une quelconque des revendications 1 à 5, caractérisée en ce qu'elle comprend également un alcool linéaire ou ramifié comportant de 1 à 6 atomes de carbone, à titre de co-solvant.

**7.** Composition selon l'une quelconque des revendications 1 à 6, caractérisée en ce qu'elle comprend également au moins un co-solvant de formule (I)

$$R_1\text{-}A\text{-}(CH_2)_x(O)_y\text{-}R_2 \qquad (I)$$

dans laquelle:

- $R_1$ représente un groupe méthyle, éthyle ou isopropyle,
- $R_2$ représente l'hydrogène ou un groupe méthyle, éthyle ou isopropyle,
- ou bien $R_1$ et $R_2$ représentent ensemble un groupement $-CH_2-CH_2-$dans la formule (I) ci-dessus cyclisée,
- x = 0, 1 ou 2
- y =0 ou 1
- A est -O- ,

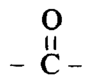 ou 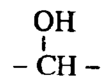 ,

ledit co-solvant ayant un point d'ébullition compris entre environ 25°C et 200°C à la pression atmosphérique.

**8.** Composition selon l'une quelconque des revendications 1 à 7, caractérisée en ce que la matière active est choisie parmi des agents nettoyants, des agents cirants, des agents désinfectants, des agents séquestrants, des ions alcalino-terreux, des agents anti-taches, des agents détartrants, des solvants, des filtres anti-UV, des agents conditionneurs, des parfums, des résines coiffantes, ou des agents insecticides ou herbicides.

**9.** Composition selon l'une quelconque des revendications 1 à 8, caractérisée en ce qu'elle est propulsée par un gaz comprimé choisi parmi le groupe constitué par l'air, l'azote, l'oxygène, l'hélium, les gaz rares et leurs mélanges.

**10.** Composition selon la revendication 9, caractérisée en ce que la pression à l'intérieur de l'aérosol est comprise entre 400 et 1200 kPa, de préférence entre 600 et 1000 kPa.

**11.** Utilisation d'une composition selon l'une quelconque des revendications 1 à 10 en tant que produit d'entretien, produit phytosanitaire, produit pharmaceutique ou produit cosmétique.

Office européen des brevets

# RAPPORT DE RECHERCHE EUROPEENNE

Numero de la demande
EP 93 40 2121

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | DE-A-39 41 572 (WELLA)<br>* page 2, ligne 37 - page 3, ligne 56; revendications 1-13; exemple 3 *<br>--- | 1-11 | A61K7/00<br>A61K7/15<br>C09K3/30 |
| X | WO-A-90 05774 (IMAGINATIVE RESEARCH ASSOCIATES)<br>* page 4, ligne 14 - page 8, ligne 15; revendications 1-13; exemples 2,9,11-13,20 *<br>--- | 1-7,11 | |
| X | US-A-4 772 427 (DAWSON ET AL.)<br>* colonne 3, ligne 13 - colonne 6, ligne 27; revendications 1-14; exemple 1 *<br>----- | 1-5,7,11 | |
| | | | DOMAINES TECHNIQUES RECHERCHES (Int.Cl.5) |
| | | | A61K |

Le présent rapport a été établi pour toutes les revendications

| Lieu de la recherche | Date d'achèvement de la recherche | Examinateur |
|---|---|---|
| LA HAYE | 1 Décembre 1993 | Willekens, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)